# EUROPÄISCHE PATENTSCHRIFT

(11) **EP 0 627 006 B1**
(45) Veröffentlichungstag und Bekanntmachung des Hinweises auf die Patenterteilung: **02.05.1997**
(21) Anmeldenummer: 93905242.9
(22) Anmeldetag: 12.02.1993
(51) Int. Cl.: C12N 15/62, C12N 15/74, C07K 7/00, C07K 14/00

(54) **HYBRID-DNA, DIE EINE UNTEREINHEIT EINES BAKTERIELLEN TOXINS MIT EINER HAEMOLYSIN KODIERT**
HYBRID DNA THAT CODES FOR A SUBUNIT OF A BACTERIAL TOXIN WITH A HEMOLYSIN
ADN HYBRIDE QUI CODE UNE UNITE SECONDAIRE D'UNE TOXINE BACTERIENNE AVEC UNE HEMOLYSINE

(30) Priorität: 17.02.1992 DE 4204737; 16.06.1992 DE 4219696
(43) Veröffentlichungstag der Anmeldung: 07.12.1994
(73) Patentinhaber: Gesellschaft für Biotechnologische Forschung mbH (GBF), D-38124 Braunschweig (DE)
(72) Erfinder: BRAHMBHATT, Himanshu, D-3300 Braunschweig (DE); SU, Guo-fu, D-3300 Braunschweig (DE); WEHLAND, Jürgen, D-3300 Braunschweig (DE); TIMMIS, Kenneth, N., D-3300 Braunschweig (DE)
(74) Vertreter: Boeters, Hans Dietrich, Dr.
(86) Internationale Anmeldenummer: EP9300348
(87) Internationale Veröffentlichungsnummer: WO9316186

(56) Entgegenhaltungen:
- JOURNAL OF BACTERIOLOGY, Bd. 174, Nr. 1, Januar 1992, Seiten 291-297; DALTON R. MCWHINNEY ET AL.: 'Separable domains define target cell specificities of an RTX Hemolysin from Actinobacillus pleuropneumoniae'
- INFECTION AND IMMUNITY, Bd. 59, Nr. 11, November 1991, WASHINGTON, US, Seiten 4212-4220; CHRISTIANE FORESTIER ET AL.: 'Identification of RTX toxin target cell specificity domains by use of hybrid genes'
- EMBO JOURNAL, Bd. 6, Nr. 9, September 1987, EYNSHAM, OXFORD, GB, Seiten 2835-2841; N. MACKMAN ET AL.: 'Release of a chimeric protein into the medium from Escherichia coli using the C-terminal secretion signal of haemolysin'

## Beschreibung

Bakterielle Dysenterie, die durch Shigella-Arten hervorgerufen wird, ist eine ansteckende Erkrankung der Darmschleimhaut beim Menschen und anderen Primaten. *Shigella dysenteriae* 1 (Shiga's bacillus) verursacht die schwerste Form der Krankheit und kann zu ernsten Komplikationen führen, beispielsweise dem haemolytischen Uraemiesyndrom (H.U.S., wozu haemolytische Anaemie, Thrombocytopenia und akutes Nierenversagen zählen), leukämoide Reaktionen und Sepsis, insbesondere bei Kindern. Es ist gegenwärtig für eine große Pandemie verantwortlich, die den indischen Subkontinent, Teile von Afrika, Südostasien, China und Teile von Lateinamerika heimsucht.

Eins der unterscheidenden Merkmale von *S. dysenteriae* 1 ist die Bildung großer Mengen von Shigatoxin, einem der potentesten Cytotoxine, die bekannt sind. Obgleich Shigatoxin eine Vielzahl von Zelltypen abtötet, ist es besonders aktiv gegenüber vaskulären Endothelialzellen, was vermutlich für die schweren Komplikationen verantwortlich ist, die mit Infektionen durch Shiga's bacillus verbunden sind. Anti-Dysenterie-Impfstoffe sollten daher einen Schutz nicht nur gegen den Organismus selbst, sondern auch gegen Shigatoxin vorsehen.

Shigaartige Toxine spielen auch eine Rolle bei anderen Infektionen des Menschen und der Tiere. Einige Serotypen von Escherichia coli, beispielsweise O157:H7, produzieren haemorrhagische Colitis, eine afebrile wässerige Diarrhö, die in blutige Diarrhö übergeht, und bei Versuchstieren lassen sich Symtome von haemorrhagischer Colitis dadurch reproduzieren, daß das gereinigte Toxin allein verabreicht wird. Oft sind mit haemorrhagischer Colitis schwere Komplikationen, wie H.U.S. verbunden, die gleichfalls mit Shiga-bacillus-Infektionen zusammenhängen. Shigaartiges Toxin produzierende *E. coli* Serotypen verursachen gleichfalls ernsthafte Erkrankungen, wie die ödemerkrankung des Schweins bei landwirtschaftlich gehaltenen Tieren, was zu beträchtlichen wirtschaftlichen Verlusten führt.

Es besteht daher ein dringendes Bedürfnis, Impfstoffe zur Anwendung sowohl auf dem Human- als auch auf dem Tiersektor gegen Shiga und verwandte Toxine zu entwickeln.

Shiga- und shigaartige Toxine sind zweiteilige (bipartite Moleküle), die sich aus zwei verschiedenen Typen von Untereinheiten aufbauen, der A-Untereinheit (Mr = 32.000) und der B-Untereinheit (Mr = 7.700), die nicht-kovalent in einem stöchiometrischen Verhältnis der Untereinheiten von etwa einer A-Kette zu fünf B-Ketten assoziiert sind. Die A-Untereinheit ist gegenüber eucaryotischen Zellen toxisch, und die B-Untereinheit besitzt eine Rezeptor-Bindungsstelle, die die Aufnahme des Toxins in Zellen ermöglicht. Daher sollte eine wirksame Schleimhaut- und Serum-Anti-Toxin-Immunantwort gegen die B-Untereinheit das Eindringen des Toxins in intestinale Epithelialzellen und vaskuläre Endothelialzellen verhindern, um eine ernste Erkrankung auszuschliessen.

Jedoch ist eine hochgradige Expression von Fremdpolypeptiden in Impfstoffstämmen mit mehreren Nachteilen belastet; Beipiele sind:
(i) Das Fusionieren von heterologen Polypeptiden mit Trägermolekülen, beispielsweise dem LamB-Protein, ist oft schwierig, da für die Größe des passierenden bzw. Passsagier-Polypeptids (passenger polypeptide), das stabil eingesetzt werden kann, eine Grenze gegeben ist (Charbit et al. 1988).
(ii) Hocheffektive Expression heterologer Polypeptide im Cytoplasma führt oft zum Abbau des Proteins und der Bildung von Einschlußkörpern.
(iii) Die Fusionierung von Fremdpolypeptiden mit Trägermolekülen, wie ß-Galactosidase, im Hinblick auf eine Expression im Cytoplasma führt gleichfalls oft zu Toxizität gegenüber der Zelle, was die Impfstoffstämme unbrauchbar macht.

Es besteht daher ein Bedürfnis, ein geeignetes Expressionssystem zu entwickeln, mit dem sich große heterologe Polypeptide stabil in großer Ausbeute exprimieren lassen, ohne daß Einschlußkörper gebildet werden oder Toxizität gegenüber den Impfstoffstämmen auftritt.

Unlängst ist ein genetisches System entwickelt worden, um passierende bzw. Passagierproteine (passenger proteins) aus *E. coli* in das Medium mit Hilfe einer Fusion an die 23 kD große C-terminale Signaldomäne von Haemolysin (HlyA) abzugeben; Mackman et al. 1987. Die Haemolysin-Gene sind in einem Operon (*hly* C, A, B, D) organisiert, und das HlyC-Protein ist in die postranslationale Aktivierung des 107 kD großen HlyA-Proteins zu seiner aktiven Form verwickelt. Der Export- bzw. Ausschleusungsmechanismus benutzt ein spezifisches Sekretionssignal in den letzten 50 C-terminalen Aminosäuren des HlyA und einen membrangebundenen Translokationskomplex, der sich aus HlyB und HlyD aufbaut (Wagner et al. 1983), sowie mindestens ein Gastprotein, das kleinere Außenmembranprotein TolC (Wandersman und Delepelaire 1990), das das HlyA-Molekül direkt zum Medium ohne periplasmatische Zwischenstufe transportiert (Gray et al. 1986 und Koronakis et al. 1989).

Dieser Stand der Technik sei wie folgt noch näher erläutert. Um oral zu verabreichende Impfstoffe gegen Infektionen zu entwickeln, die auf *S. dysenteriae* 1 zurückgehen, wurden attenuierte bzw. abgeschwächte *Salmonella-*Hybridstämme konstruiert, die das O-Antigen von *S. dysenteriae* 1 exprimieren (Mills et al. 1988 und Mills & Timmis 1988), um O-Antigen-spezifische, lokalisierte Schleimhautimmun-Antworten zu stimulieren. Eine notwendige zusätzliche Komponente der Hybridimpfstoffstämme ist der Einschluß einer in geeigneter Weise exprimierten Shigatoxin-Untereinheit B, um eine Immunantwort hervorzurufen, die die biologische Aktivität des Toxins neutralisiert, um den Schweregrad der Erkrankung herabzusetzen.

Die Expression von Fremdpolypeptiden, die an Trägermoleküle fusioniert sind, wie beispielsweise das LamB-Protein, leidet an dem Nachteil, das es für das Passagier-Polypeptid, das stabil eingesetzt werden kann, eine Größenbeschränkung gibt (Charbit et al. 1988). Für größere Polypeptide können alternative Maßnahmen vorgesehen werden, beispielsweise eine Fusion an den N-terminalen Bereich der β-Galactosidase (Jacob et al. 1985, Brown et al. 1987. Unlängst ist ein genetisches System für die Ausschleusung von Passagier-Proteinen aus *E. coli* in das Medium mit Hilfe einer Fusion an die 23 kD große C-terminale Signaldomäne von Haemolysin (HlyA) (Mackman et al. 1987) mit dem Anliegen entwickelt worden, rekombinante Proteine zu reinigen.

Zu verweisen ist auf Hybrid-Toxine, die aus einem Fragment des Hämolysins (C-terminales Ende) und einem Fragment eines zweiten Toxins bestehen und die gentechnisch hergestellt worden sind; vergl. McWhinney et al. in J. Bacteriology, 174 (1982) 291-297 und Forrestier & Welch in Infection Immunity, 59 (1991) 4212-4220.

Das Cytotoxin, also Haemolysin, wird durch uropathogene Stämme von *Escherichia coli* (Welch et al. 1981) sekretiert und in das Medium ohne Vermittlung einer N-terminalen Signal-secA/secY-Route ausgeschleust bzw. transloziert (Felmlee et al. 1985 und Blight et al. 1990). Die Haemolysin-Gene sind in einem Operon (*hly* C, A, B, D) organisiert, und das HlyC-Protein ist in eine posttranslationale Aktivierung des 107 kD großen HlyA-Proteins zur cytotoxischen Form einbezogen. Der Exportmechanismus benutzt ein spezifische Sekretionssignal in den letzten 50 C-terminalen Aminosäuren des HlyA und einen membrangebundenen Translokationskomplex, aufgebaut aus HlyB und HlyD (Wagner et al. 1983), sowie mindestens ein Gastprotein, das kleinere Außenmembran-Protein TolC (Wandersman & Delepelaire 1990), das das HlyA-Molekül direkt in das Medium ohne periplasmatische Zwischenstufe transportiert (Gray et al. 1986 und Koronakes et al. 1983).

Die der Erfindung zugrundeliegende Aufgabe wird nun durch eine Hybrid-DNA gelöst, bei der eine DNA-Struktur, die ein Fragment eines bakteriellen Toxins kodiert, mit einem C-terminalen Fragment einer HlyA (Hämolysin) kodierenden DNA-Struktur fusioniert ist, wobei diese Hybrid-DNA dadurch gekennzeichent ist, daß es sich bei dem Fragment des bakteriellen Toxins um die Untereinheit B von Shigatoxin oder von shigaartigen Toxinen handelt.

Bevorzugte Ausführungsformen und weitere Gegenstände der Erfindung ergeben sich aus den Ansprüchen 2 bis 10.

Erfindungsgemäß wurde nun also die Shigatoxin-Untereinheit B mit dem 23 kD großen C-Terminus von *E. coli* Haemolysin A (HlyA) fusioniert und festgestellt, daß das Fusionsprotein von dem attenuierten Antigen-Trägerstamm *Salmonella typhimurium* aroA⁻ SL3261 ausgeschleust wird. Die Expression der Genfusion wurde unter der Kontrolle eines modifizierten synthetischen β-Lactamase-Promotors (konstitutive Expression) und der Kontrolle des *in vivo* induzierbaren Aerobactin-Promotors untersucht. Orale und intraperitoneale Immunisierung von Mäusen mit *Salmonella typhimurium aro*A-(SL3261)-Hybridstämmen führte zu einer Stimulierung von Immunantworten auf die Untereinheit B, die für Schleimhaut spezifisch ist, und von Serumantikörpern-Antworten.

Erfindungsgemäß kann damit erstmals berichtet werden, daß ein ausgewähltes Antigen (candidate antigen) von einem Antigenträger-Impfstoffstamm der Gattung *Salmonella* für antigenspezifische Immunantworten ausgeschleust wird. Dieses System weist die folgenden Vorteile auf:
(i) Es können größere Polypeptide an die Haemolysinexportmaschine solange fusioniert werden, wie das heterologe Polypeptid keine Transferstopsequenzen ("stop transfer" sequences) kodiert.
(ii) Da sich das Fusionsprotein nicht in den Zellen anhäuft, können sehr hohe Expressionsniveaus erreicht werden, ohne daß es zu einer Toxizität für den Impfstamm kommt.

Das erfindungsgemäße System ist daher auch zur Expression von shigaartigen Toxinen, wie SLT-II und SLT-IIv, geeignet, um starke Immunantworten gegen ihre rezeptorbindende Untereinheit zu liefern, und ferner ist es auch für andere bakterielle Toxine von Schleimhauterregern geeignet.

Erfindungsgemäß wurde also das Shigatoxin-Protein der Untereinheit B mit dem 23 kD großen C-Terminus von HlyA fusioniert, um das Untereinheit B/HlyA-Fusionsprotein in das extrazelluläre Medium zu exportieren. Derartige Hybridproteine (chimeric proteins) wurden in *Salmonella typhimurium aro*A-Antigenträger-Impfstamm SL3261 (Hoseith & Stocker 1981) zum Einsatz für orale und intraperitoneale Immunisierungen exprimiert, um zu ermitteln, ob Immunantworten stimuliert werden konnten, die für die Untereinheit B spezifisch sind.

Erfindungsgemäß wurde also die Shigatoxin-Untereinheit B mit dem 23 kD großen C-Terminus von *E. coli*-Haemolysin A (HlyA) fusioniert und von dem attenuierten Antigenträgerstamm *Salmonella typhimurium aroA*^{*-*} SL3261 exportiert. Die Expression des Hybridproteins wurde unter der Kontrolle eines modifiziertes synthetischen β-Lactamase-Promotors (konstitutive Expression) und unter der Kontrolle eines *in vivo* induzierbaren Aerobactin-Promotors untersucht. Auch wurde der Einfluß der Plasmidkopien-Anzahl unter Verwendung von Mediumkopier- und Hochleistungskopier-Plasmidvektoren untersucht. Es wurde eine Stimulierung von für die Untereinheit B spezifischen Antikörper-Antworten in Mäusen nach oraler und intraperitonealer Immunisierung mit Hybridstämmen SL3261 durchgeführt, um drei unterschiedliche Arten der Untereinheit-B-Expression zu untersuchen.
(i) Cytoplasmatische Hochleistungsexpression der Untereinheit B.
(ii) Das cytoplasmatisch exprimierte Untereinheit B/β-Galactosidase-Fusionsprotein.
(iii) Das Untereinheit B/HlyA-(23 kD C-Terminus)-Fusionsprotein, ausgeschleust in die extrazelluläre Umgebung.

Erfindungsgemäß wurde die Expression von Shigatoxin-Untereinheit B/Haemolysin-A-(C-Terminus)-Fusionsproteinen unter vier verschiedenen Bedingungen untersucht:
(a) Die *Stx*B/C-T:*hly*A-Genfusion war unter der Kontrolle eines *in* *vivo* induzierbaren Aerobactin-Promotors, der entweder vom Plasmidvektor pBR322 (Kopienzahl etwa 30 bis 40/Zelle/Generation) oder
(b) vom PLasmid pUC18 (Kopienzahl etwa 70/Zelle/Generation) getragen wurde und
(c) die *stx*B/C-T:*hly*A-Fusion war unter der Kontrolle eines konstitutiven, modifizierten, synthetischen β-Lactamase-Promotors, der eine Expression auf moderatem Niveau lieferte, wobei die Fusionen gleichfalls entweder mit Hilfe des Plasmids pBR322 oder
(d) des Plasmids pUC18 durchgeführt wurden. Damit war eine Untersuchung der Promotorstärke und der Plasmidkopienzahl im Hinblick auf die Stabiliät einer Expression des StxB/C-T:HlyA-Fusionsproteins möglich.
(e) Ferner wurde eine Vergleichsanalyse durchgeführt, bei der die Untereinheit B auf hohem Niveau in Cytoplasma exprimiert wurde und ferner ein β-Galactosidase-Fusionsprotein in Cytoplasma exprimiert wurde.

Untersuchungen des Stands der Technik unter Einsatz der Haemolysin-Exportmaschinerie zur Sekretion heterologer Polypeptide wurden in *E. coli* durchgeführt. Es war jedoch nicht bekannt, ob derartige Fusionsproteine auch von *Salmonella typhimurium* exportiert werden könnten. Die erfindungsgemäßen Expressionsstudien wurden gleichzeitig sowohl mit *E. coli* K-12 und dem Antigenträger-Impfstamm *S. typhimurium aro*A*-* SL3261 durchgeführt.

Die Vergleichsanalyse zeigte, daß die Fusionsproteine sowohl von *E. coli* K-12 als auch von *S. typhimurium-aroA-*Stämmen exportiert werden konnten, was nahelegte, daß die Haemolysin-Exportmaschinerie in *S. typhimurium* funktionierte. Rekombinante Plasmide auf Basis von pUC18 erwiesen sich jedoch als für die Zellen toxisch, wobei ein gewisser Abbau des Fusionsproteins festgestellt werden konnte. Die Bakterienzellen, die die Plasmide beherbergten, konnten nicht bis zu einer Zelldichte von mehr als 10⁷ Lebendzellen/ml gezüchtet werden. Andererseits waren die Plasmide auf Basis von pBR322 stabil; es gab keinen Hinweis auf einen Abbau des Fusionsproteins, wobei eine Lebendzelldichte von 10⁹ Zellen/ml leicht zu erzielen war. Bei früheren Untersuchungen, bei denen verschiedene Regionen der Shigatoxin-Untereinheit B in die der Bakterienzelloberfläche ausgesetzte Schleife des LamB-Proteins fusioniert wurden, wurde festgestellt, daß nur kurze Untereinheit B-Polypeptide stabil unter Verwendung von LamB als Trägermolekül exprimiert werden konnten. Die vollständige Untereinheit B (69 Aminosäuren), die in das LamB-Expressionssystem fusioniert worden war, erwies sich gegenüber den Wirtsbakterien als toxisch und bildete große intrazelluläre Aggregate. Die Haemolysin-Exportmaschinerie scheint daher dann geeignet zu sein, wenn größere Polypeptide als Fusionsproteine exprimiert werden müssen, sofern das eingefügte Polypeptid keine übertragungs-Stoppsequenzen ("stop transfer" sequences) aufweist.

Die S. typhimurium aroA-Hybridstämme, die die Untereinheit B als
(i) Cytoplasma-Protein exprimieren oder
(ii) fusioniert an β-Galactosidase und cytoplasmatisch exprimieren oder
(iii) fusioniert mit HlyA (C-Terminus) in die extrazelluläre Umgebung ausschleusen, wurden zur oralen und intraperetonealen Immunisierung von Mäusen verwendet, um zu untersuchen, welches Expressionssystem sich bei der Stimulierung von für die Untereinheit B spezifischen Antikörperantworten als am wirksamsten erwies. Die Ergebnisse zeigen, daß signifikante Antikörperantworten auf die Untereinheit B mit allen drei Systemen erreicht werden konnten. Jedoch scheint eine hochgradige Expression des gewünschten Antigens (candidate antigen) für das Wirtsbakterium nachteilig zu sein, so daß eine moderate Expressionsrate zweckmäßiger erscheint. Eine konstitutive Expression, beispielsweise mit dem synthetischen β-Lactamase-Promotor, oder ein in vivo induzierbares System, beispielsweise mit dem Aerobactin-Promotor, lieferte bessere Ergebnisse bezogen auf die Stabilität des Wirtsbakteriums.

Die Serumantworten waren im allgemeinen stärker als die Schleimhautantikörper-Antworten. Ein möglicher Grund für diese Beobachtung könnte darin zu sehen sein, daß die Gewinnung bzw. Isolierung von Antikörpern aus dem Darm erschwert ist. Untereinheit B/β-Galactosidase-Fusionsproteine zeigten beträchtlich höhere β-Galactosidase-Antworten, was möglicherweise eine höhere Epitopdichte bei dem viel größeren β-Galactosidase-Polypeptid (107 kD) im Vergleich zur Untereinheit B (7,7 kD) widerspiegelt.

Nachstehend wird die Erfindung durch Figuren und experimentelle Daten näher erläutert.

### Figurenbeschreibung

### Fig. 1:

Plasmidkonstruktionen für die Expression von StxB/C-T:HlyA-Fusionsproteinen. Zur besseren übersichtlichkeit sind nur die relevanten Teile der Einfügungen der Plasmide dargestellt.

### Fig. 2:

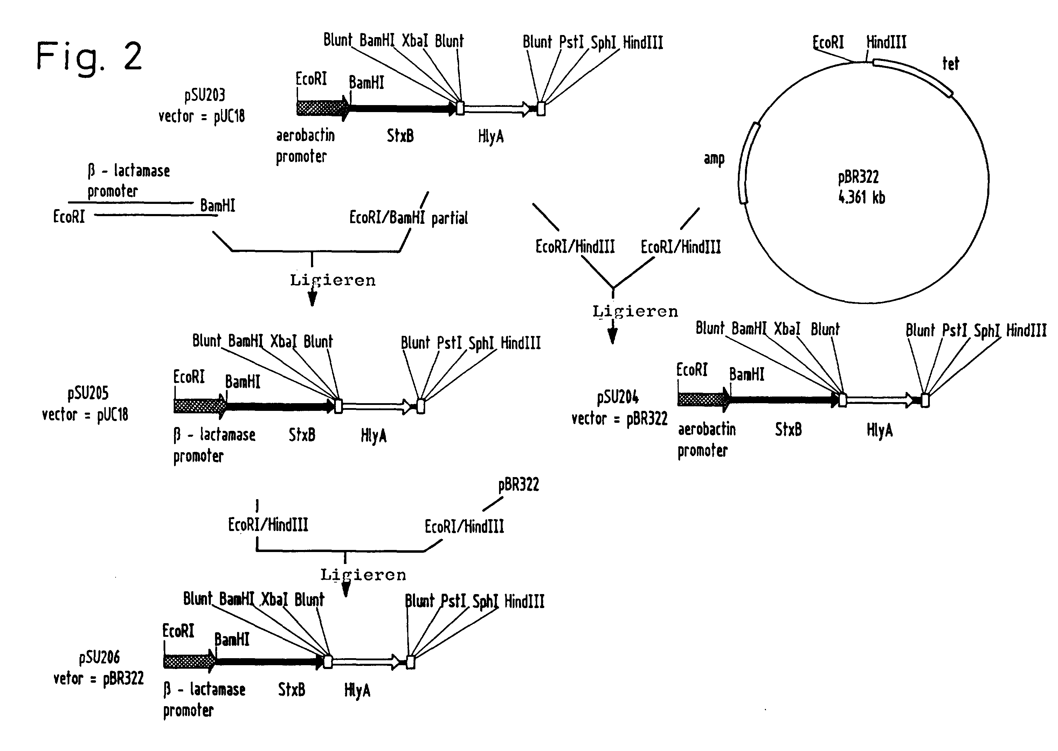

Western-Blott-Analyse der Expression von Shigatoxin-Untereinheit B/Haemolysin-A-(C-Terminus)-Fusionsproteinen. Die Bereichte der Untereinheit B der Fusionsproteine wurden durch für die Untereinheit B spezifische monoklonale Antikörper StxBMb1 ermittelt.
(A) Bahn 1, *E. coli* JM101/pLG612+pLG575 (Gesamtzellextrakt); Bahn 2, Kulturüberstand der Zellen von Bahn 1; Bahn 3, JM101/pSU204+pLG575 (Aerobaktin-Promotor/*stx*B/C-T:*hly*A/pBR322; Gesamtzellextrakt); Bahn 4, Kulturüberstand der Zellen von Bahn 3; Bahn 5, JM101/pSU206+pLG575 (MS β-Lactamase-Promotor/*stx*B/C-T:*hly*A/pBR322; Gesamtzellextrakt); Bahn 6, Kulturüberstand der Zellen von Bahn 5.
(B) Man vergleiche Fig. 2A mit der Ausnahme, daß der Wirtsstamm *Salmonella typhimurium aroA*^{*-*} SL3261 ist.
(C) Bahn 1, SL3261/pLG612+pLG575 (Gesamtzellextrakt); Bahn 2, Kulturüberstand der Zellen von Bahn 1; Bahn 3, SL3261/pSU203+pLG575 (Aerobactin-Promotor/*stx*B/C-T:*hly*A/pUC18; Gesamtzellextrakt); Bahn 4, Kulturüberstand der Zellen von Bahn 3; Bahn 5, SL3261/pSU205+pLG575 (MS β-Lactamase-Promotor/*stx*B/C-T :*hly*A/pUC18; Gesamtzellextrakt); Bahn 6, Kulturüberstand der Zellen von Bahn 5.

### Fig. 3:

Schleimhaut- und Serumantikörperantworten in Mäusen nach oraler und intraperotenealer Immunisierung mit verschiedenen *S. typhimurium aro*A-(SL3261)-Hybridstämmen.

### Fig. 3.1:

Antworten auf die Untereinheit B nach Immunisierung mit SL3261/pSU108 (cytoplasmatische Hochleistungsexpression der Untereinheit B) und SL3261/pJL503 (negative Kontrolle).

### Fig. 3.2:

Antikörperantworten auf Untereinheit B und β-Galactosidase nach Immunisierung mit SL3261/pSU207 (Untereinheit B/β-Galactosidase-Fusionsprotein, cytoplasmatisch exprimiert; Expression der Kontrolle des Aerobactin-Promotors) und SL3261/pcon1 (Plasmidvektor, der *lac*Z unter der Kontrolle des Aerobactin-Promotors exprimiert; negative Kontrolle auf Antworten auf die Untereinheit B).

### Fig. 3.3:

Antikörperantworten auf Untereinheit B nach Immunisierung mit SL3261/pLG575/pSU204 (Expression von *stx*B/C-T::*hly*A unter der Kontrolle des Aerobactin-Promotors), SL3261/pLG575/pSU206 (Expression von *stx*B/C-T::*hly*A unter der Kontrolle des MS-β-Lactamase-Promotors) und SL3261/pLG575/pLG612 (Expression von C-T::*hly*A unter der Kontrolle des *lac*-Promotors; negative Kontrolle auf Antworten auf die Untereinheit B).

### MATERIALIEN UND METHODEN.

### Bakterienstämme, Plasmide und Medien:

*E. coli-*Stamm JM101 (F-, traD36, *lac*I^{q}, (*lac*Z)M15, *pro*AB*, sup*E*, thi, lac-pro*AB; vergleiche Yanisch-Perron et al. 1985) wurde als Empfänger für alle rekombinanten Plasmide verwendet. Die *Salmonella-typhimurium-aro*A*-Mutante* SL3261 (Hoseith & Stocker 1981) und der restriktionsnegative *S.-typhimurium-*Stamm SL5283 wurden von B.A.D. Stocker (Stanford Univ. Schl. of Medicine, USA) zur Verfügung gestellt. Die Plasmide pLG612 (trägt den 23 kD großen C-Terminus von *hly*A und einen Teil des N-Terminus von *hly*B unter der Kontrolle des *lac*-Promotors, der mit IPTG induziert werden kann) und pLG575 (Chloramphenicol-resistentes Plasmid), das die *hly*B- und *hly*D-Gene trägt, geklont in das *tet*-Resistenzgen des Plasmids pACYC184 (Mackman et al. 1985) wurden von Dr. Holland (Univ. of Leicester, U.K.) zur Verfügung gestellt. Das Plasmid pSU201 (Guo-fu Su et al. 1992) trägt das *stx*B-Gen unter der Kontrolle des Aerobaction-Promotors und die Plasmide pBR32 (Bolivar et al. 1977) und pUC18 (Yanisch-Perron et al. 1985) wurden als Klonierungsvektoren für allgemeine Zwecke verwendet. Die folgenden Plasmide wurden von Guo-Fu Su und Mitarbeitern beschrieben (1992); (i) Plasmid pSU108 (trägt das *stx*B-Gen unter der Kontrolle von lambda-P_{L}- und lambda-P_{R}-Promotoren, die wäremeinduzierbar sind);
(ii) Plasmid pJLA503 (Hochleistungsexpressions-Plasmid; Schauder et al. 1987);
(iii) Plasmid pSU207 (trägt die *stx*B/*lac*Z-Genfusion unter der Kontrolle eines Aerobactin-Promotors);
(iv) Plasmid pcon 1 (trägt das *lac*Z-Gen unter der Kontrolle eines Aerobactin-Promotors; Lorenzo et al. 1987) und
Luria-Brühe und Luria-Agar (Miller 1972) wurden als vollständige Medien für routinemäßiges Wachstum aller Stämme verwendet; falls erforderlich, wurden Bakterienwachstumsmedien mit Antibiotika Ampicillin (100 µg/ml) oder Chloramphenicol (30µg/ml) supplementiert. Restriktionsendonuclease, T4-DNA-Ligase, DNA-Polymerase (Klenow Enzym) und alle anderen Enzyme wurden entweder von Böhringer GmbH (Mannheim, Deutschland) oder von New England Biol-abs, Inc., Beverly, Mass.) bezogen und entsprechend den Angaben des Herstellers eingesetzt.

DNA-Manipulationen: DNA-Präparation und genetische Manipulationen wurden nach Standardprotokollen durchgeführt (Maniatis et al. 1982); Plasmid DNA-Transformation von Bakterienzellen wurden gemäß Hanahan (1983) vorgenommen.

SDS-Polyacrylamid Gel-Elektrophorese: Diskontinuierliche Natrium Dodecyl Sulfat-Polyacrylamid-Gel-Elektrophorese (SDS-Page) wurde gemäß Schägger & Jagow (1987) vorgenommen. Zuvor markierte SDS-Page-Molekulargewichtsmarker waren entweder von Bio-Rad (97,4, 66,2, 45,0, 31,0, 21,5 und 14,4 kD) oder von Sigma (84,0, 58,0, 48,5, 36,5, 26,6 kD); man vergleiche die **Figuren**.

Western-Blott-Analyse von Gesamtzellextrakten und Kulturüberstandfraktionen: Bakterienzellen von übernachtkulturen (1 ml) wurden durch Zentrifugieren gesammelt. Zu den überstandsfraktionen (etwa 900 µl) gab man 100 µl 100-proz. TCA (Tri-Chlor-Essigsäure) um extrazelluläre Proteine auszufällen. Nach sorgfältigem Mischen wurde die Probe bei 4 °C 30 min. lang inkubiert und 15 min. lang zentrifugiert, wobei der überstand verworfen wurde. Das Protein Pellet (Kulturüberstandsfraktion) wurde in 20 µl Puffer resuspendiert (16 µl 1M Tris-base, 2 µl 10x Crack Puffer [1x Crack Puffer: 60 mM Tris-HCl pH 6,8, 1-proz. SDS, 1-proz. 2-Mercaptoethanol, 10-proz. Glycerin 0,01 mg Bromphenol blau] 2µl Glycerin). Das Bakterien Pellet (Gesamtzellfraktion) wurde in 50 µl Puffer resuspendiert. Der überstand und die Gesamtzellfranktionen wurden auf 100 °C 10 min. lang erhitzt, bevor man 20 µl-Proben auf SDS-page gab. Nach Elektrophorese wurden die Gele auf Nitrozellulose blottiert; die Anwesenheit von Shiga toxin-Untereinheit B/HlyA-Fusionsproteinen wurde durch für die Untereinheit B spezifische monoklonale Antikörper StxBMb1 nachgewiesen (Guo-Fu Su et al. 1992).

Immunisierung und Sammeln von Seren und Darmwäschen: 8 bis 10 Wochen alte weibliche BALB/c Mäuse (vier Mäuse pro Immunisieung) wurden oral und intraperetoneal praktisch gemäß Guo-Fu sun und Mitarbeitern (1992) immunisiert. Innocula zur Immunisierung wurden folgendermaßen vorgesehen.

Es wurden über Nacht Kulturen des *S. typhimurium-aro*A-Stammes SL3261, die entweder das Plasmid pSU108 oder das Plasmid pJLA503 (Negativkontrolle) trugen, frisch in Luria-Brühe mit einem Gehalt an Ampicillin kultiviert, bis ein OD₆₀₀-Wert von 0,7 erreicht wurde. Die Zellen wurden für 45 min. auf 42 °C gebracht, um die lambda PL- und P_{R}-Promotoren zu induzieren. Die Kulturen wurden zweimal in steriler normaler Kochsalzlösung gewaschen und in einem geeigneten Volumen Kochsalzlösung bis zu einer Endkonzentration von 10¹¹ CFU/ml resuspendiert. 0,1 ml der Zellsuspension wurden für die orale Immunisierung verwendet. Ferner wurden die Zellen auf 10⁶ CFU/ml verdünnt, wobei 0,1 ml Zellen für die intraperitoneale Immunisierung verwendet wurden.

SL3261-Stämme, die entweder das Plasmid pcon1 (Negativkontrolle) oder das Plasmid pSU207 trugen) wurden gleichfalls wie vorstehend wachsengelassen, bis der OD₆₀₀-Wert 0,3 erreichte. Man gab 2,2'-Bipyridyl hinzu (Endkonzentration 100 µM, zum Induzieren des Aerobactin-Promotors); man ließ die Zellen weiter 3 h lang wachsen. Die Kulturen wurden gewaschen, resuspendiert und wie vorstehend zum Immunisieren verwendet. Man ließ SL3261/pLG575-Stämme, die entweder das Plasmid pLG612 (Negativkontrolle), pSU204 oder pSU206 trugen, wie vorstehend in Luria-Brühe (ergänzt mit Ampicillin und Chloramphenicol) wachsen, bis der OD₆₀₀-Wert 1,0 (für Plasmid pSU206) bzw. 0,3 (für Plasmid pLG612 und pSU204) erreichte. Zellen mit Plasmid pLG612 wurden mit IPTG (Enkonzentration 1 mM) induziert, und Zellen mit Plasmid pSU204 wurden mit 2,2'-Bipyridyl (Endkonzentration 100 µM) induziert. SL3261/pLG575/pLG612-Kulturen ließ man ferner 45 min. lang und SL3261/pLG575/pSU204-Kulturen ferner 3 h lang wachsen. Die Zellen wurden gewaschen, resuspendiert und zum Immunisieren wie vorstehend verwendet.

ELISA: Proben für ELISA wurden reihenweise in einer mit Phosphat gepufferten Kochsalzlösung (pH 7,2) verdünnt. Bestimmungen der Anti-Untereinheit B und der Anti-Glactosidase wurden dadurch durchgeführt, daß man zuvor Mikrotiterplatten mit 1 µg gereinigter Untereinheit B (Guo-fu Su et al. 1992) bzw. 1 µg gereinigter B-Galatosidase (von Sigma Chemical Co.) überzog. Für Serumbestimmungen von IgG + IgM wurde IgG + IgM (peroxidase conjugated goat anti-mouse IgG + IgM; von Dianova) verwendet; Schleimhaut-IgA wurde dadurch bestimmt, daß man IgA (peroxidase conjugated goat anti-mouse IgA; von Southern Biotechnology Inc.) verwendete. Die Ergebnisse wurden mit einem Bio-Rad-Microplate-Reader (Modell 3550) gelesen.

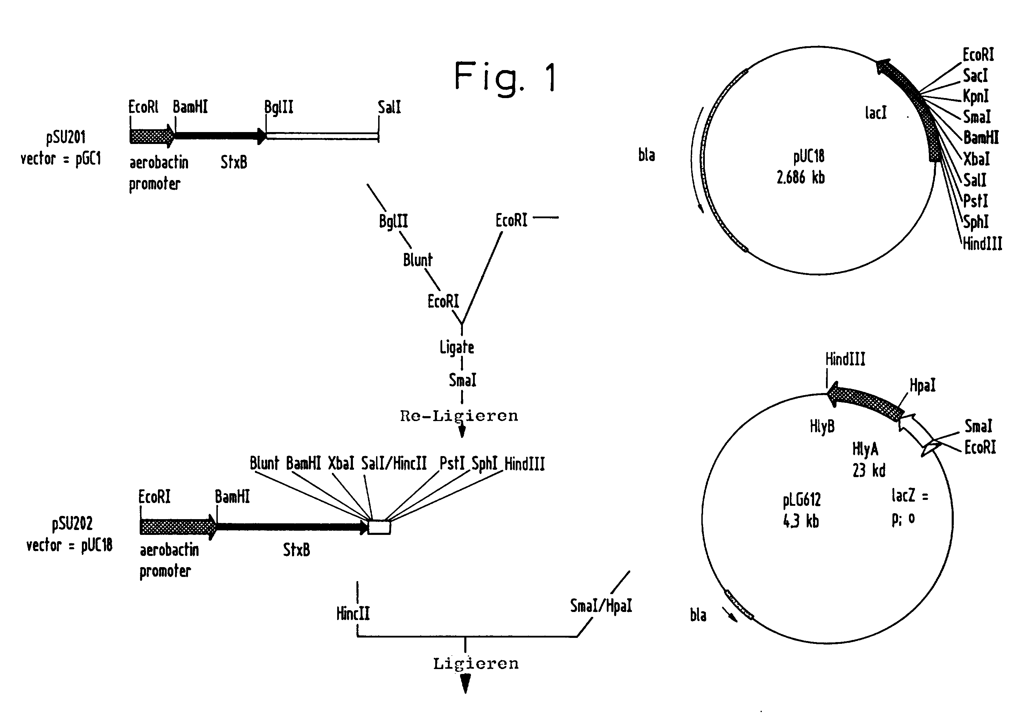

Plasmidkonstruktion zur Expression von HlyA-(C-Terminus) /Untereinheit B-Fusionsproteinen: Das *Eco*RI/*Bgl*II-Fragment des Phagemids pSU201, das *stx*B unter der Kontrolle des Aerobactin-Promotors trug, wurde in die *Eco*RI/*Smal*I-Stellen des Plasmids pUC18 subkloniert, wobei man Plasmid pSU202 erhielt; **Figur 1.** Die C-terminale 23 kD große Region von *hly*A (als C-T:*hly*A bezeichnet) und etwa 50 Basenpaare des N-terminalen Bereichs von *hly*B auf einem *Sma*I/*Hpa*-Fragment wurden in die *Hinc*II-Stelle des Plasmids pSU202 eingesetzt. Das resultierende Plasmid pSU203 (**Figur 1**) trägt das *stx*B-Gen, das im Leserahmen mit C-T:*hly*A fusioniert ist.

Das *Eco*RI/*Hind*III-Fragment des Plasmids pSU203, das die Aerobactin-Promotor/*stx*B/C-T:*hly*A-Kassette trägt, wurde in die *Eco*RI-*Hind*III-Stelle des Plasmids pBR322 subkloniert, wobei man Plasmid pSU204 erhielt; **Figur 1**. Das *Eco*RI/*Bam*HI-Fragment von Plasmid pSU203, das den Aerobactin-Promotor trägt, wurde durch den modifizierten synthetischen β-Lactamase-Promotor ersetzt (als MS β-Lactamase-Promotor bezeichnet). Diese Konstruktion führte zum Plasmid pSU205 (**Figur 1**), das eine *Eco*RI*-Hind*III-Kassette trägt, die die MS-β-Lactamase-Promotor/*stx*B/C-T:*hly*A-Region umfaßt, die in die *Eco*RI/*Hind*III-Stellen des Plasmids pBR322 eingesetzt werden konnte, wobei man Plasmid pSU206 erhielt; **Figur 1.**

Die Plasmide pSU203 und pSU204 (die die Aerobactin-Promotor/*stx*B/C-T:*hly*A-Kassette im Vektor pUC18 bzw. pBR322 tragen) und die Plasmide pSU205 und pSU206 (die die MS-β-Lactamose-Promotor/*stx*B/C-T:*hly*A-Kassette im Vektor pUC18 bzw. pBR322 tragen), wurden in *E. coli* K 12- und *S. typhimurium-aro*A-*Stämme* SL3261 transformiert, die Plasmid pLG575 trugen. Vor der Transformation bei *S. typhimurium aro*A- SL3261, wurden die Plasmide zuerst durch den restriktionsnegativen Stamm *S. typhimurium* SL5283 passiert.

Western-Blott-Analyse von StxB/C-T:HlyA-Fusionsproteinen in E. coli K 12 und Salmonella-typhimurium-aroA-Stamm SL3261: Das Pellet und die überstandsfraktionen der Bakterienzellen, die die rekombinanten Plasmide bzw. die Vergleichsplasmide beherbergten, wurden an SDS-PAGE eine Elektrophorese und eine Western-Blott-Analyse auf Nitrocellulose unterworfen. Man ermittelte die Fusionsproteine, indem man den für die Untereinheit B spezifischen monoklonalen Antikörper SStxBMb1 einsetzte (**Fig. 2**). Man stellte fest, daß das StxB/C-T:HlyA-Fusionsprotein in die extracelluläre Umgebung bei allen untersuchten Stämmen exportiert wurde; **Figuren 2A, 2B und 2C;** Bahnen 4 und 6. Das StxB/C-T:HlyA-Fusionsprotein, das vom Aerobactin-Promotor exprimiert wird, ist 7 kD größer als das gleiche Fusionsprotein, das vom MS-β-Lactamase-Promotor exprimiert wird, da das *Eco*RI/*Bam*HI-Aerobactin-Promotor-Fragment im Plasmid pSU201 (**Figur 1**) ferner einen Teil des *iuc*A-Gens trägt (das erste Gen des Aerobactin Operons), das ein 7 kD großes Polypeptid kodiert (Lorenzo et al. 1987).

Die Menge des exprimierten Fusionsproteins war viel größer in Plasmiden auf Basis von pUC18 als in Vektoren auf Basis von pBR322; man vergleiche **Figuren 2B und 2C**; Bahnen 4 und 6. Jedoch führte die höhere Expressionsrate auch zu einem gewissen Abbau des Fusionsproteins (**Figur 2C;** Bahnen 4 und 6), wobei sich Toxizität gegenüber den Wirtszellen ergab. Da es nicht möglich war, diese Bakterien zu einer Lebendzelldichte von mehr als 10⁷/ml heranwachsen zu lassen. Die Konstrukte auf Basis von pBR322 waren stabil, wobei man die Zellen bis zu 10⁹ Lebendzellen/ml heranwachsen lassen konnte.

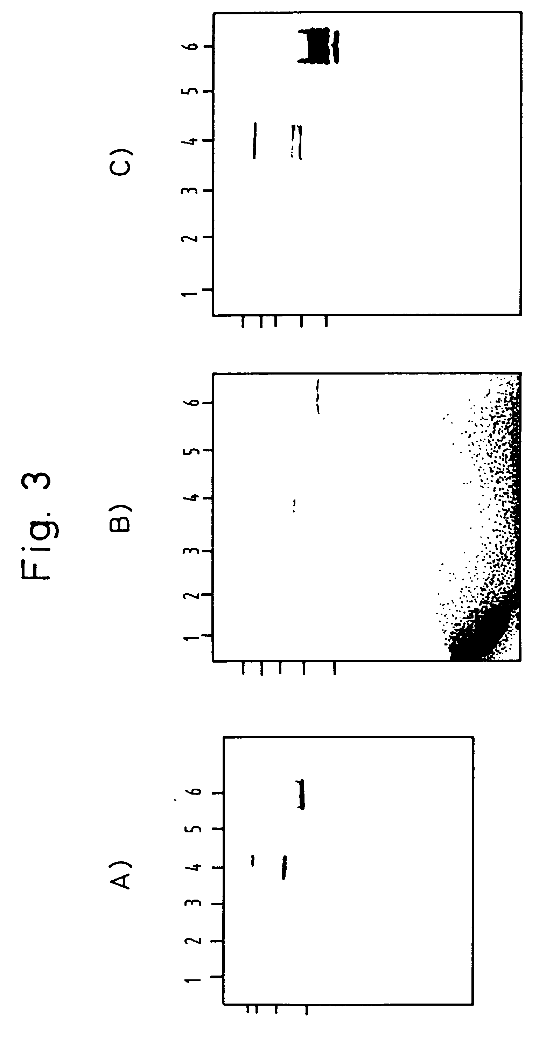

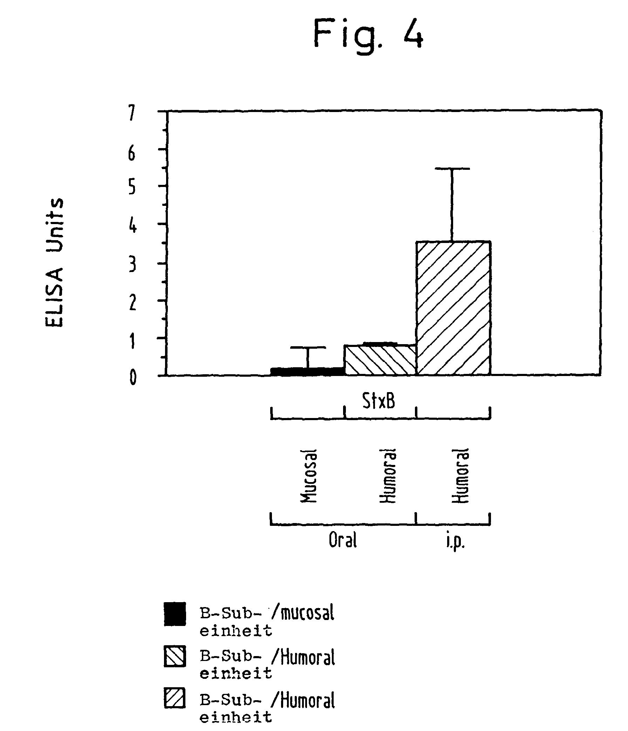

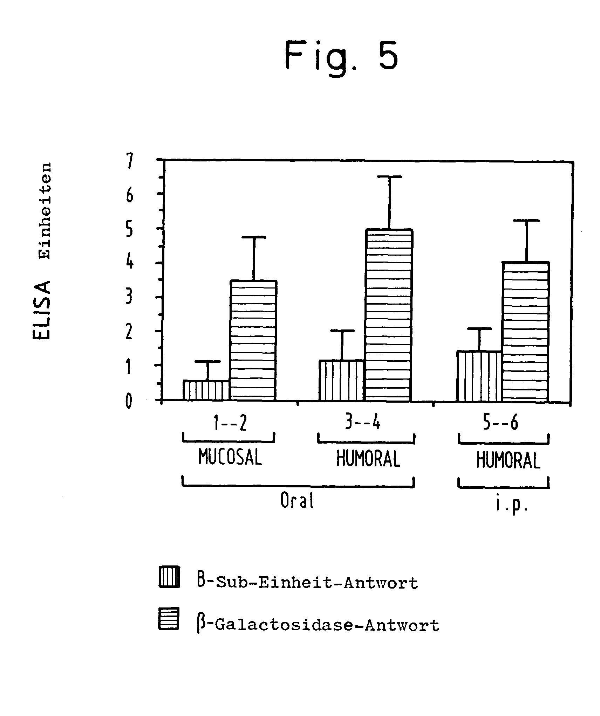

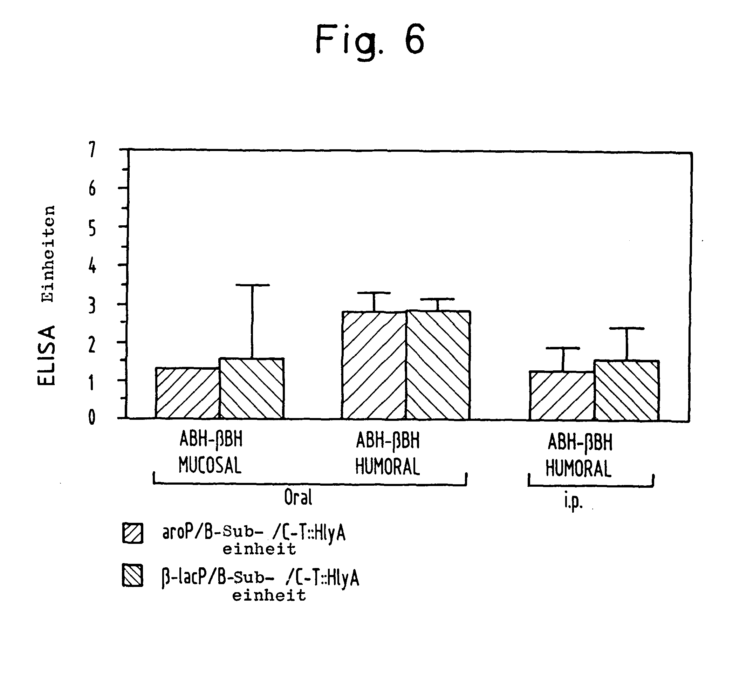

Analyse von für die Untereinheit B spezifischen Immunantworten in Mäusen nach oraler und interperitonealer Immunisierung mit dem Salmonella-typhimurium-aroA-Hybridstamm SL3261: Man analysierte *Salmonella typhimurium-aro*A-Stämme (SL3261) die die Untereinheit B in drei verschiedenen Formen exprimieren, zum Stimulieren von für die Untereinheit B spezifischen Immunantworten in Mäusen nach oraler bzw. interperitonealer Immunisierung. Die getesteten Expressionssysteme umfaßten folgende Expressionen:
(1) Cytoplasmatische Hochleistungsexpression der Untereinheit B (Plasmid pSU108; guo-fu Su et al. 1992);
(ii) cytoplasmatische Expression eines Untereinheit B/β-Galactosidase-Fusionsproteins (Plasmid pSU207; Guo-fu Su et al. 1992);
(iii) Untereinheit B/C-T::HlyA-Fusionsprotein, exprimiert entweder unter der Kontrolle des Aerobactin- oder des MS-β-Lactamase-Promotors (Plasmide pSU204 und pSU206). Diese Fusionsproteine wurden in den extrazellulären Darm exportiert. Man analysierte Darmflüssigkeit auch Anti-Untereinheit B-Schleimhaut-IgA- und Serumantikörper-Antworten (IgG + IgM), während man das Serum der interperitonealen Immunisierung von Mäusen auf Antikörper-Antworten (IgG + IgM) analysierte. Die Proben, die man nach Immunisierung mit *S. typhimurium-aro*A erhielt, daß das Plasmid pSU207 trug, wurde auf β-Galactosidase-Antworten untersucht. Die Ergebnisse (**Figuren 3.1, 3.2 und 3.3**) zeigten, daß in allen Fällen Signifikante für die Untereinheit B spezifischer Antikörper-Antworten sowohl im Serum als auch in der Darmflüssigkeit ermittelt werden konnten. Jedoch waren Salmonellen, die das Plasmid pSU108 trugen, instabil, und zwar vermutlich in Folge der hochgradigen Expression der rekombinanten Untereinheit B. Die Serumantikörper-Antworten waren im allgemeinen höher als die Schleimhautantikörper-Antworten. Für Plasmid pSU207 (**Fig. 3.2**) waren die Anti-β-Galactosidase Antikörper-Antworten beträchtlich höher als die für die Untereinheit B spezifischen Antikörper-Antworten.

| Lebendmaterial | Zugang |
|---|---|
| DNA für HlyA (= Hämolysin) | siehe pSU 204 = DSM 7045 oder pSU 205 = DSM 7046 |
| | |
| SLT-II (= Shiga Like Toxin II) | Stockbin et al., Infect. Immun., 50 (1985) 695-700 und 53 (1986) 135-140 |
| | |
| SLT-IIv | loc. cit. |
| | |
| β-lactamase-Promotor | siehe pSU 205 = DSM 7046 |
| | |
| Aerobactin-Promotor | siehe pSU 204 = DSM 7045 |
| | |
| pBR 322 | Boehringer Mannheim |
| | |
| pUC 18 | Boehringer Mannheim |
| | |
| S. t. aroA SL3261 | Hosieth & Stocker, Nature, 291 (1981) 238-239 |

### REFERENCES.

Blight MA, Holland IB (1990) Structure and function of haemolysin B, P-glycoprotein and other members of a novel family of membrane translocators. Molec. Microbiol. 4: 873-880.

Bolivar F, Rodriguez RL, Betlach MC, Boyer HW (1977) Construction and characterization of new cloning vehicles. I. Ampicillin-resistant derivatives of plasmid pMB9. Gene 2: 75-93.

Brown A, Hormaeche CE, De Hormaeche RD, Winther M, Dougan G, Maskell DJ, Stocker BAD (1987) An attenuated *aro*A *Salmonella typhimurium* vaccine elicits humoral and cellular immunity to cloned β-galactosidase in mice. J. Infect. Dis. 155: 86-92.

Charbit A, Molla A, Saurin W, Hofnung M. (1988) Versatility of a vector to express foreign polypeptides at the surface of Gram-negative bacteria. Gene 70: 181-189.

Felmlee T, Pellet S, Lee E-Y, Welch R (1985) *Escherichia coli* haemolysin is released extracellularly without cleavage of a signal peptide. J. Bacteriol. 163: 88-93.

Gray L, Mackman N, Nicaud J-M, Holland IB (1986)............ Mol. Gen. Genet. 205: 127-133.

Guo-fu Su, Brahmbhatt HN, Wehland J, Rohde M, Timmis KN (1992) Construction of stable LamB/Shiga toxin B-subunit hybrids, analysis of expression in *Salmonella typhimurium aro*A-strains and stimulation of B-subunit specific mucosal and serum antibody responses: Potential anti-Shiga toxin component of dysentery vaccine............

Hanahan D (1983) Studies in transformation of *Escherichia coli* with plasmids. J. Mol. Biol. 166: 577-580.

Hoiseth SK, Stocker B.A.D. (1981) Aromatic-dependent *Salmonella lyphimurium* are non-virulent and effective as live vaccines. Nature 291: 238-239.

Koronakis V, Kornakis E, Hughes C (1989) Isolation and analysis of the C-terminal signal directing the export of *Escherichia coli* haemolysin protein across both bacterial membranes. EMBO J. 8: 595-605.

Lorenzo VD, Wee S, Herrero M, Neilands JB (1987) Operator sequences of the aerobactin operon of plasmid ColV-K30 binding the ferric uptake regulation *(fur)* repressor. J. Bacteriol. 169: 2624-2630.

Maniatis T, Fritsch EF, Sambrook J(1982) Molecular Cloning. A Laboratory Manual. Cold Spring Harbor, NY.

Mackman N, Nicaud J-M, Gray L, Holland IB (1985) Genetical and functional organization of the *Escherichia coli* haemolysin determinant 2001. Mol. Gen. Genet. 201: 282-288.

Mackman N, Baker K, Gray L, Haigh R, Nicaud J-M, Holland IB (1987) Release of a chimeric protein into the medium from *Escherichia coli* using the C-terminal secretion signal of haemolysin. EMBO J. 6: 2835-2841.

Mills SD, Sekizaki T, Gonzalez-Carrero MI, Timmis KN (1988) Analysis and genetic manipulation of *Shigella* virulence determinants for vaccine development. Vaccine 6: 116-122.

Mills SD, Timmis KN (1988) Genetics of O-antigen polysaccharide biosynthesis in *Shigella* and vaccine development. In: Cabello FC, Pruzzo C (Eds) Bacteria, Complement and the Phagocytic Cell. Springer Verlag, Berlin, Heidelberg, pp 21-39.

Schägger H, Jagow GV (1987) Tricine-Sodium Dodecyl Sulfate-Polyacrylamide Gel electrophoresis for the separation of proteins in the range from 1 to 100 kDa. Anal. Biochem. 166: 368-379.

Schauder B, Blöcker H, Frank R, McCarthy JEG (1987) Inducible expression vectors incorporating the *Escherichia coli atp*E translation initiation region. Gene 52: 279-283.

Smith HW (1963) The haemolysins of *Escherichia coli.* J. Pathol. Bacteriol. 85: 197-211.

Wagner W, Vogel M, Goebel W (1983) Transport of haemolysin across the outer membrane of *Escherichia coli* requires two functions. J. Bacteriol. 154: 200-210.

Wandersman C, Delepelaire P (1990) *Tol*C, and *Escherichia coli* outer membrane protein required for haemolysin secretion. Proc. Natl. Acad. Sci. USA 87: 4776-4780.

Welch RA, Patchen-Dellinger E, Minschew B, Falkow S (1981) Haemolysin contributes to virulence of extraintestinal *E. coli* infections. Nature 294: 665-667.

Yanisch-Perron C, Vieira J, Messing J (1985) Improved M13 phage cloning vectors and host strains: nucleotide sequences of the M13mp18 and pUC19 vectors. Gene 33: 103-119.

## Patentansprüche

1. Hybrid-DNA, bei der eine DNA-Struktur, die ein Fragment eines bakteriellen Toxins kodiert, mit einem C-terminalen Fragment einer HlyA (Hämolysin) kodierenden DNA-Struktur fusioniert ist, dadurch **gekennzeichnet**, daß es sich bei dem Fragment des bakteriellen Toxins um die Untereinheit B von Shigatoxin oder shigaartigen Toxinen handelt.

2. Hybrid-DNA nach Anspruch 1, dadurch **gekennzeichnet**, daß es sich bei shigaartigen Toxinen um SLT-II oder SLT-IIv handelt.

3. Hybrid-DNA nach Anspruch 1 oder 2, dadurch **gekennzeichnet,** daß den beiden kodierenden DNA-Strukturen ein konstitutiver Promotor oder ein unter in-vivo-Bedingungen induzierbarer Promotor vorgeschaltet ist.

4. Hybrid-DNA nach Anspruch 3, dadurch gekennzeichnet, daß den beiden kodierenden DNA-Strukturen ein Promotor zur Expression in einem Salmonella-Stamm, insbesondere *S.* typhimurium, beispielsweise *S.* t. aroA⁻ SL3261, vorgeschaltet ist.

5. Hybrid-DNA nach Anspruch 4, **gekennzeichnet** durch einen β-Lactamase-Promotor vom Wildtyp oder als Modifikation des Wildtyps, wobei der Promotor synthetisch hergestellt sein kann oder nicht.

6. Hybrid-DNA nach Anspruch 4, **gekennzeichnet** durch einen Aerobactin-Promotor.

7. Plasmid, **gekennzeichnet** durch eine Hybrid-DNA gemäß einem der vorhergehenden Ansprüche.

8. Wirtsstamm, **gekennzeichnet** durch ein Plasmid gemäß Anspruch 7, insbesondere Salmonella-Stamm, insbesondere *S.* typhimurium-Stamm, beispielsweise *S.* t. aroA⁻ SL3261.

9. Oligohybridpeptid, **gekennzeichnet** durch die Untereinheit B von Shigatoxin oder von shigaartigen Toxinen, die mit einem C-terminalen Fragment von HlyA fusioniert ist.

10. Impfstoff, bestehend aus oder umfassend das Oligohybridpeptid gemäß Anspruch 9.

## Claims

1. Hybrid DNA, wherein a DNA structure coding for a fragment of a bacterial toxin is fused with a C-terminal fragment of a DNA structure coding for HlyA (haemolysin), *characterized* in that the fragment of the bacterial toxin is subunit B of Shigatoxin or Shiga-like toxins.

2. Hybrid DNA according to claim 1, *characterized* in that the Shiga-like toxins are SLT-II or SLT-IIv.

3. Hybrid DNA according to claim 1 to 2, *characterized* in that a constitutive promotor or a promotor which can be induced under in vivo conditions, is provided in front of both coding DNA structures.

4. Hybrid DNA according to claim 3, *characterized* in that a promotor for the expression in a *Salmonella* strain, especially *S. typhimurium,* for example *S. t.* aroA⁻ SL3261, is provided in front of both coding DNA structures.

5. Hybrid DNA according to claim 4, *characterized* by a β-Lactamase promotor of the wild type or as a modification of the wild type, wherein the promotor can be provided synthetically or not.

6. Hybrid DNA according to claim 4, *characterized* by an Aerobactin promotor.

7. Plasmid, *characterized* by a hybrid DNA according to any of the preceding claims.

8. Host strain, *characterized* by a plasmid according to claim 7, especially a *Salmonella* strain, especially *S. typhimurium,* for example *S. t.* aroA⁻ SL3261.

9. Oligohybrid peptide, *characterized* by subunit B of Shiga toxin or Shiga-like toxins, being fused with a C-terminal fragment of HlyA.

10. Vaccine, consisting of or comprising the oligohybrid peptide according to claim 9.

## Revendications

1. ADN hybride pour lequel une structure d'ADN codant un fragment d'une toxine bactérienne a été fusionnée avec un fragment C-terminal d'une structure d'ADN codant la HlyA (hémolysine), caractérisé en ce que le fragment de la toxine bactérienne est la sous-unité B de la shigatoxine ou de toxines apparentées.

2. ADN hybride conforme à la revendication 1, caractérisé en ce que lesdites toxines apparentées sont la SLT-II ou la SLT-IIv.

3. ADN hybride conforme à la revendication 1 ou 2, caractérisé en ce que les deux structures d'ADN codantes son précédées d'un promoteur constitutif ou d'un promoteur inductible dans les conditions *in vivo.*

4. ADN hybride conforme à la revendication 3, caractérisé en ce que les deux structures d'ADN codantes sont précédées d'un promoteur permettant leur expression dans une souche *Salmonella,* en particulier *S. typhimurium,* par exemple S. t. aroA⁻ SL3261.

5. ADN hybride conforme à la revendication 4, caractérisé par un promoteur de la β-lactamase de type sauvage ou d'une variante de celui-ci, le promoteur pouvant être préparé par synthèse ou non.

6. ADN hybride conforme à la revendication 4 caractérisé par un promoteur de l'aérobactine.

7. Plasmide caractérisé en ce qu'il contient un ADN hybride conforme à une des revendications précédentes.

8. Souche hôte caractérisée en ce qu'elle héberge un plasmide conforme à la revendication 7, en particulier une souche *Salmonella,* en particulier *S. typhimurium,* par exemple S. t. aroA⁻ SL3261.

9. Oligopeptide hybride caractérisé en ce qu'il contient la sous-unité B de la shigatoxine ou de toxines apparentées, laquelle sous-unité a été fusionnée avec un fragment C-terminal de la HlyA.

10. Vaccin constitué de ou comprenant l'oligopeptide hybride conforme à la revendication 9.
